(19) Europäisches Patentamt
European Patent Office
Office européen des brevets

(11) Veröffentlichungsnummer: **0 053 714** B1

(12) # EUROPÄISCHE PATENTSCHRIFT

(45) Veröffentlichungstag der Patentschrift: **29.08.84**

(51) Int. Cl.³: **C 07 C 125/08**, C 07 C 127/19

(21) Anmeldenummer: **81109191.7**

(22) Anmeldetag: **29.10.81**

(54) **Solvatisierte Salze von 2,4-Dinitrophenyl-cyanamiden, Verfahren zu ihrer Herstellung und ihre Verwendung.**

(30) Priorität: **10.12.80 DE 3046490**

(43) Veröffentlichungstag der Anmeldung: **16.06.82 Patentblatt 82/24**

(45) Bekanntmachung des Hinweises auf die Patenterteilung: **29.08.84 Patentblatt 84/35**

(84) Benannte Vertragsstaaten: **BE CH DE FR GB IT LI**

(56) Entgegenhaltungen:
**DE - A - 2 204 479**
**DE - A - 2 855 882**

**BEILSTEINS "Handbuch der organischen Chemie", 4. Auflage, 2. Ergänzungswerk, Band 12 1950, SPRINGER VERLAG, Berlin, Seite 411, "N-Cyan-2,4-dinitro-anilin, 2,4-Dinitro-phenylcyanamid"**

(73) Patentinhaber: **HOECHST AKTIENGESELLSCHAFT, Postfach 80 03 20, D-6230 Frankfurt am Main 80 (DE)**

(72) Erfinder: **Arndt, Otto, Dr., Frankfurter Strasse 38, D-6238 Hofheim am Taunus (DE)**
Erfinder: **Papenfuhs, Theodor, Dr., Heinrich-Bleicher-Strasse 40, D-6000 Frankfurt am Main 50 (DE)**
Erfinder: **Tronich, Wolfgang, Dr., Adolf-Guckes-Weg 5, D-6239 Eppstein/Taunus (DE)**

**EP 0 053 714 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft Verbindungen der Formel I

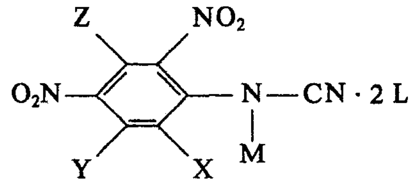

in der X Wasserstoff, Fluor, Chlor oder Brom, Y und Z Wasserstoff, niederes Alkyl oder niederes Alkoxy, M Natrium, Kalium oder ein Äquivalent Calcium und L ein dipolar-aprotisches Lösemittel bedeuten.

Bevorzugt sind Verbindungen der Formel I, in der X Wasserstoff, insbesondere Chlor, Y und Z Methyl, Methoxy, insbesondere Wasserstoff, M Natrium, insbesondere ein Äquivalent Calcium und L Tetramethylensulfon, insbesondere N-Methylpyrrolidon oder Dimethylsulfoxid bedeuten.

Die Erfindung betrifft weiterhin ein Verfahren zur Herstellung der Verbindungen I aus Verbindungen der Formel II

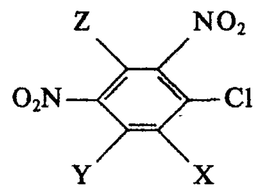

in der X, Y und Z die vorstehend genannten Bedeutungen haben, und Cyanamid in einem polaren Lösemittel, das dadurch gekennzeichnet ist, daß man das Cyanamid als Metallcyanamid der Formel III

$$M_2N-CN$$

in der M die vorstehend genannte Bedeutung hat, und als polares Lösemittel ein dipolar-aprotisches Lösemittel einsetzt, das nach der Umsetzung weitgehend abgetrennt wird, worauf dem Rückstand ein niedermolekularer aliphatischer Alkohol zugesetzt und das ausgeschiedene Produkt isoliert wird.

Die Erfindung betrifft weiterhin die Verwendung der Verbindungen der Formel I zur Herstellung von Dinitrophenylharnstoffen der Formel IV

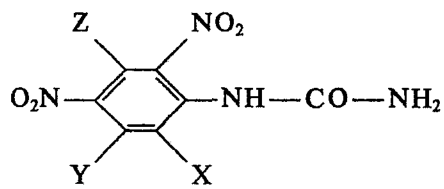

in der X, Y und Z die vorstehend genannten Bedeutungen haben, durch Hydrolyse. Die Harnstoffe werden hierbei in hoher Reinheit erhalten.

Aus J. pract. Chem. [2] 110 (1925) 300 ist es bekannt, 2,4-Dinitrochlorbenzol mit einem großen Überschuß von Cyanamid in wäßrigem Äthanol zu 2,4-Dinitrophenylcyanamid umzusetzen und dieses mit Salzsäure auszufällen. Durch Erhitzen mit alkoholischer Salzsäure wird daraus der 2,4-Dinitrophenylharnstoff erhalten.

Aus der US-PS 3 979 453 ist es bekannt, N-substituierte 3-Chlor-2,6-dinitro-4-trifluormethylaniline mit Cyanamid in Gegenwart einer starken Base in inerten Lösemitteln wie Äthanol, Dioxan oder Tetrahydrofuran umzusetzen. Die Reaktionslösung wird nach Abkühlen auf Eiswasser gegossen und das ausgefallene Produkt aus Äthanol und Wasser umkristallisiert.

In der DE-OS 2 855 882 ist beschrieben, daß man 2,4-Dinitro-1-chlorbenzol mit Cyanamid in wäßrigem Medium zum 2,4-Dinitrophenyl-cyanamid umsetzen und ohne Isolierung durch saure Hydrolyse zum 2,4-Dinitrophenylharnstoff verseifen kann.

Im Gegensatz zu diesen bekannten Herstellungsmethoden geht das erfindungsgemäße Verfahren nicht vom freien Cyanamid aus, sondern von den bei den üblichen technischen Verfahren erhaltenen Metallsalzen, insbesondere dem Calciumcyanamid, das vorteilhaft in Form des technischen Kalkstickstoffs eingesetzt wird. Hierbei fallen die Verbindungen der Formel I an, die in kristalliner Form isoliert werden können und bei ihrer Verseifung die entsprechenden Harnstoffe in hoher Reinheit liefern.

Im folgenden werden bevorzugte Ausgestaltungen der Erfindung näher erläutert.

Die Umsetzung erfolgt vorteilhaft bei Temperaturen von etwa 25 bis etwa 90° C, insbesondere etwa

50 bis etwa 70° C.

Als dipolares Lösemittel kommen Acetonitril, Dimethylformamid, Dimethylacetamid, Tetramethylensulfon und insbesondere Dimethylsulfoxid und N-Methylpyrrolidon in Betracht.

Die Umsetzungsgeschwindigkeit kann durch Zusatz geringer Mengen von Cyanamid erhöht werden, das zweckmäßig in der handelsüblichen kristallinen Form eingesetzt wird. Die Menge beträgt etwa 0,1 bis etwa 0,25 Mol pro Mol Metallcyanamid, das seinerseits zweckmäßig in einem Überschuß von etwa 10 bis etwa 80% eingesetzt wird. Bevorzugt werden insgesamt pro Mol Verbindung der Formel II etwa 1,5 Mol Cyanamide (Summe aus Metallcyanamid und freiem Cyanamid) gewählt.

Da unter den Reaktionsbedingungen die Verbindung der Formel II mit Wasser das entsprechende Phenol bilden kann, wird vorzugsweise unter Ausschluß von Wasser und — im Gegensatz zu den bekannten Verfahren — ohne Basenzusatz gearbeitet.

Der Ablauf der Reaktion kann durch Entnahme von Proben und deren dünnschichtchromatographische Auswertung verfolgt werden, wobei das Verschwinden der Ausgangskomponente der Formel II das Ende der Umsetzung anzeigt.

Nach erfolgter Umsetzung wird die Reaktionsmischung von festen Bestandteilen (überschüssiges Metallcyanamid, beim Einsatz von Kalkstickstoff außerdem Calciumoxid und Kohle) befreit und das dipolar-aprotische Lösemittel weitgehend abgetrennt, vorzugsweise durch Vakuum-Destillation. Der Destillationsrückstand wird mit dem niederen aliphatischen Alkohol versetzt, wobei sich das solvatisierte Salz der Formel I abscheidet. Vorteilhaft wird etwa die 4fache Gewichtsmenge Isopropanol, bezogen auf das Ausgangsmaterial der Formel II, zugesetzt. Das kristallin ausgeschiedene Salz der Formel I kann in dieser Form weiterverarbeitet werden, wird jedoch zweckmäßig abgetrennt, z. B. abfiltriert, und mit einem niedermolekularen Alkohol, insbesondere dem zur Fällung verwendeten Alkohol, gewaschen und dann entweder in dieser Form weiterverarbeitet oder vorzugsweise getrocknet, da man auf diese Weise zu besonders reinen Harnstoffen gelangt.

Die Verseifung der Verbindungen der Formel I zu den Harnstoffen der Formel IV erfolgt im sauren Medium. Zweckmäßig wird hierzu das Salz der Formel I in wäßriger Mineralsäure gelöst und die Lösung durch Zusatz von Wasser soweit verdünnt, daß das freie Cyanamid ausfällt. Dieses wird dann in wäßriger Suspension bei Temperaturen von 15 bis 80°C, bevorzugt 50 bis 70°C verseift und der entsprechende 2,4-Dinitrophenylharnstoff durch Filtration isoliert.

Der entstandene Harnstoff der Formel IV kann unmittelbar weiterverarbeitet werden. Zweckmäßig wird er mit Wasser, Natriumhydrogencarbonatlösung bis zur neutralen Reaktion und wieder mit Wasser gewaschen. Das so erhaltene Feuchtprodukt kann ohne Trocknung weiterverarbeitet werden, beispielsweise gemäß DE-OS 2 855 883 zum entsprechenden 5-Amino-benzimidazolon. Der Harnstoff kann jedoch auch getrocknet werden, beispielsweise bei etwa 60° C im Vakuum. Die 2,4-Dinitrophenylharnstoffe der Formel IV fallen in sehr reiner Form an und enthalten nur sehr geringe Mengen des entsprechenden 2,4-Dinitroanilins.

In der DE-OS 2 204 479 wird im Zuge der Beschreibung der Herstellung von Alkyl-2-benzimidazolcarbamaten, ausgehend vom o-Halogennitrobenzol, auch die Herstellung von o-Nitrocarbanilonitrilsalz beschrieben. Die solvatisierten Salze (mit 2 Mol eines dipolaren aprotischen Lösungsmittels) werden dort jedoch nicht beschrieben und fallen nach dem dort (Beispiel 1, Schritt 1), beschriebenen Verfahren auch nicht an. Es muß als überraschend erachtet werden, daß gerade die mit 2 Mol eines dipolaren aprotischen Lösungsmittels solvatisierten Salze der Formel I bei ihrer Verseifung die entsprechenden Harnstoffderivate in hoher Reinheit liefern.

In den folgenden Beispielen beziehen sich Teile und Prozentangaben auf das Gewicht, sofern nichts anderes angegeben ist.

## Beispiel 1

474 Teile (2 Mol) 1,2-Dichlor-3,5-dinitrobenzol werden in 4740 Teilen N-Methylpyrrolidon mit 343 Teilen 63%igem Kalkstickstoff (2,7 Mol) und 13 Teilen krist. 98%igem Cyanamid (0,3 Mol) 15 Stunden bei 60° C verrührt. Man trennt vom restlichen Kalkstickstoff durch Filtrieren ab und wäscht letzteren mit 200 Teilen N-Methylpyrrolidon. Die vereinigten Filtrate werden in einem Vakuum von bis zu 5 mbar bei Temperaturen bis maximal 85—87° C eingeengt. Der Destillationsrückstand wird in 1600 Teilen Isopropanol aufgenommen. Das bei 40° C auskristallisierte Calciumsalz des 6-Chlor-2,4-dinitrophenylcyanamid, solvatisiert mit 2 Mol N-Methylpyrrolidon, wird bei 10° C abfiltriert, mit Isopropanol gewaschen und bei 50° C i.V. getrocknet.

Man erhält 773 Teile Kristallisat, Fp. 114° C, entspr. einer Ausbeute von 84%. Die Elementaranalyse entspricht der Formel

$$C_{17}H_{20}ClN_6O_6Ca_{0,5} \text{ (Molgew. 459,9).}$$

Die Struktur wurde durch magnetische Kernresonanz bestätigt.

773 Teile des getrockneten Calciumsalzes ( × 2 NMP) werden in 4900 Teilen 30%iger Salzsäure bei 5—10° C gelöst. Man verdünnt mit 2000 Teilen Wasser. Das danach auskristallisierte 6-Chlor-2,4-dinitro-phenylcyanamid verseift bei 60° C zum 6-Chlor-2,4-dinitro-phenylharnstoff, der abfiltriert wird und nacheinander mit Wasser, 5%iger Natriumhydrogencarbonatlösung und wieder Wasser gewaschen wird. Man erhält nach dem Trocknen 428 Teile reinen, in Nadeln kristallisierten hellgelben 6-Chlor-2,4-dinitrophenylharnstoff, Fp. = 220—225° C, entspr. einer Ausbeute von 98%, bezogen auf Calciumsalz, und 82%, bezogen auf 1,2-Dichlor-3,5-dinitrobenzol.

Beispiel 2

474 Teile (2 Mol) 1,2-Dichlor-3,5-dinitrobenzol werden in 4740 Teilen Dimethylsulfoxid mit 343 Teilen 63%igem Kalkstickstoff (2,7 Mol) und 13 Teilen krist. 98%igem Cyanamid (0,3 Mol) 7 Stunden bei 60° C verrührt. Die Aufarbeitung erfolgt wie in Beispiel 1.

Man erhält nach dem Trocknen 752 Teile Calciumsalz des 6-Chlor-2,4-dinitro-phenylcyanamid, solvatisiert mit 2 Mol Dimethylsulfoxid, entspr. einer Ausbeute von 90%. Fp. 165° C. Die Elementaranalyse entspricht der Formel

$C_{11}H_{14}ClN_4O_6S_2Ca_{0,5}$ (Molgew. 417,9).

Die Struktur wurde durch NMR bestätigt.

Aus 752 Teilen des getrockneten Calciumsalzes ( × 2 DMSO) erhält man durch Verseifung in Salzsäure gemäß Beispiel 1 450 Teile reinen 6-Chlor-2,4-dinitrophenylharnstoff, entspr. einer Ausbeute von 97%, bezogen auf Calciumsalz, und 86%, bezogen auf 1,2-Dichlor-3,5-dinitrobenzol. Fp. = 220—225° C. Der Harnstoff bildet wie in Beispiel 1 Nadeln, die nur wenig Mutterlauge (ca. 200 Teile) festhalten und gute Filtriereigenschaften haben. Oberfläche (nach Ströhlein) ca. 0,6 $m^2/g$.

Beispiel 3

Die Ausführung erfolgt wie in Beispiel 1 bzw. 2, jedoch mit Tetramethylensulfon (TMSO) als Lösemittel. Die Reaktion in TMSO erfordert eine höhere Temperatur von 80—90° C.

Die Ausbeute entspricht den Beispielen 1 und 2.

Beispiel 4

Die Ausführung erfolgt wie in Beispiel 2, jedoch werden 900 Teile Calciumsalz, isopropanolfeucht, mit einem Trockengehalt von 752 Teilen in der Verseifung eingesetzt. Man erhält einen Harnstoff, der erheblich feinkristalliner ist als derjenige von Beispiel 2 und dessen Oberfläche (nach Ströhlein) ca. 20 $m^2/g$ beträgt.

Bei der Filtration hält der Harnstoff viel Mutterlauge zurück (ca. 900 Teile), was die Filtration verlangsamt und die Wäsche erschwert. Das Produkt ist etwas mit 6-Chlor-2,4-dinitro-anilin (nach HPLC 1—2%) verunreinigt und hat einen Schmelzpunkt von 215—220° C.

Beispiel 5

Die Ausführung erfolgt wie in Beispiel 2, jedoch werden der Salzsäure 150 Teile Isopropanol zugesetzt. Das Ergebnis ist wie bei Beispiel 2.

Beispiel 6

Die Ausführung erfolgt wie in Beispiel 2, jedoch wird das isopropanolfeuchte Calciumsalz nicht getrocknet, sondern in der 4fachen Gewichtsmenge Wasser gelöst. Die wäßrige, schwach alkalische Lösung läuft in die 30%ige Salzsäure. Der so erhaltene 6-Chlor-2,4-dinitrophenylharnstoff ist deutlich mit 6-Chlor-2,4-dinitro-anilin ( > 2%) verunreinigt.

Beispiel 7

Die Ausführung erfolgt wie in Beispiel 1, jedoch mit dem Unterschied, daß anstelle des Kalkstickstoffs 258 Teile pulverförmiges Dinatrium-Cyanamid (3 Mol) und 32 Teile krist. 98%iges Cyanamid (0,75 Mol) eingesetzt werden. Der Einsatz des Cyanamids erfolgt in 2 Anteilen, ein Viertel wird erst gegen Ende der Umsetzung zugegeben. Die Reaktion dauert bei 60° C 28 Stunden. Anschließend wird das N-Methylpyrrolidon abdestilliert. Der Destillationsrückstand wird nach Aufnahme in 1600 Teilen Iso-

propanol mit 2450 Teilen 30%iger Salzsäure vermischt, das Gemisch auf 60°C erhitzt und 3 Stunden bei dieser Temperatur nachgerührt.

Die Isolierung des 6-Chlor-2,4-dinitro-phenylharnstoffs erfolgt wie in Beispiel 1.

Beispiel 8

81 Teile 2,4-Dinitrochlorbenzol (0,40 Mol) werden in 800 Teilen Dimethylsulfoxid mit 69 Teilen 63%igem Kalkstickstoff (0,54 Mol) und 2,6 Teilen krist. 98%igem Cyanamid (0,06 Mol) 22 Stunden bei 80°C verrührt. Man trennt vom restlichen Kalkstickstoff ab und wäscht diesen mit 80 Teilen Dimethylsulfoxid. Die vereinigten Filtrate werden im Vakuum von bis zu 5 mbar bei einer Temperatur bis maximal 70°C eingeengt. Der Destillationsrückstand wird in 300 Teilen Isopropanol aufgenommen. Das bei 47°C auskristallisierende Calciumsalz des 2,4-Dinitro-phenylcyanamid, solvatisiert mit 2 Mol Dimethylsulfoxid, wird bei 10°C abfiltriert, mit Isopropanol gewaschen und bei 50°C im Vakuum getrocknet.

Man erhält 128 Teile Kristallisat vom Fp. = 113—115°C, entspr. einer Ausbeute von 84% d. Th. Die Elementaranalyse entspricht der Formel

$C_{11}H_{15}N_4O_6S_2Ca_{0,5}$ (Molgew. 383,4)

Die Struktur wurde durch magnetische Kernresonanz bestätigt.

**Patentansprüche**

1. Verbindungen der Formel I

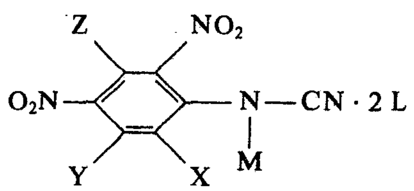

in der X Wasserstoff, Fluor, Chlor oder Brom, Y und Z Wasserstoff, niederes Alkyl oder niederes Alkoxy, M Natrium, Kalium oder ein Äquivalent Calcium und L ein dipolar-aprotisches Lösemittel bedeuten.

2. Verbindungen nach Anspruch 1, in denen X Wasserstoff oder Chlor, Y und Z Wasserstoff, Methyl oder Methoxy, M Natrium oder ein Äquivalent Calcium und L Tetramethylensulfon, N-Methylpyrrolidon oder Dimethylsulfoxid bedeuten.

3. Verbindungen nach Anspruch 2, in denen X Chlor, Y und Z Wasserstoff und M ein Äquivalent Calcium bedeuten.

4. Verfahren zur Herstellung der Verbindungen nach Anspruch 1 bis 3 aus Verbindungen der Formel II

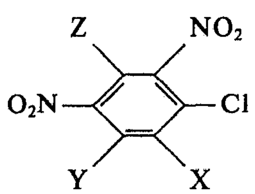

in der X, Y und Z die in Anspruch 1 genannten Bedeutungen haben, und Cyanamid in einem polaren Lösemittel, dadurch gekennzeichnet, daß man das Cyanamid als Metallcyanamid der Formel III

$M_2N-CN$

in der M die in Anspruch 1 genannte Bedeutung hat, als polares Lösemittel ein dipolar-aprotisches Lösemittel einsetzt, das nach der Umsetzung weitgehend abgetrennt wird, worauf dem Rückstand ein niedermolekularer aliphatischer Alkohol zugesetzt und das ausgeschiedene Produkt isoliert wird.

5. Verfahren nach Anspruch 4, dadurch gekennzeichnet, daß die Umsetzung bei 25 bis 90°C, insbesondere 50 bis 70°C durchgeführt wird.

6. Verfahren nach Anspruch 4 und 5, dadurch gekennzeichnet, daß das Reaktionsmedium wasserfrei ist.

7. Verfahren nach Anspruch 4 bis 6, dadurch gekennzeichnet, daß das Metallcyanamid im Überschuß eingesetzt wird.

8. Verfahren nach Anspruch 4 bis 7, dadurch gekennzeichnet, daß dem Reaktionsgemisch Cyanamid zugesetzt wird.

9. Verwendung der Verbindungen nach Anspruch 1 bis 3 zur Herstellung von Dinitrophenylharnstoffen der Formel IV

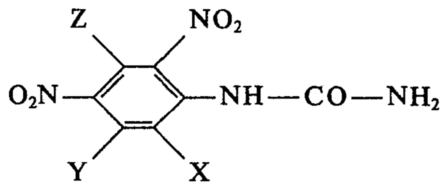

in der X, Y und Z die in Anspruch 1 genannten Bedeutungen haben, durch saure Hydrolyse.

10. Verwendung gemäß Anspruch 9, dadurch gekennzeichnet, daß aus den Verbindungen nach Anspruch 1 bis 3 zunächst das entsprechende Cyanamid freigesetzt wird.

**Claims**

1. A compound of the formula I

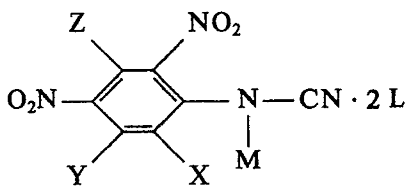

in which X denotes hydrogen, fluorine, chlorine or bromine, Y and Z denote hydrogen, lower alkyl or lower alkoxy, M denotes sodium, potassium or one equivalent of calcium and L denotes a dipolar aprotic solvent.

2. A compound as claimed in claim 1, in which X denotes hydrogen or chlorine, Y and Z denote hydrogen, methyl or methoxy, M denotes sodium or one equivalent of calcium and L denotes tetramethylene sulfone, N-methylpyrrolidone or dimethyl sulfoxide.

3. A compound as claimed in claim 2, in which X denotes chlorine, Y and Z denote hydrogen and M denotes one equivalent of calcium.

4. A process for the preparation of compounds as claimed in any one of claims 1—3 from compounds of the formula II

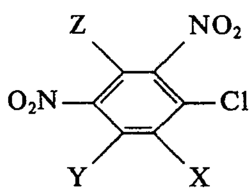

in which X, Y and Z have the meanings mentioned in claim 1, and cyanamide in a polar solvent, which process comprises using the cyanamide in the form of a metal cyanamide of the formula III

$M_2N-CN$

in which M has the meaning mentioned in claim 1, and using a dipolar aprotic solvent as the polar solvent, which is almost completely removed after the reaction, whereupon a low molecular weight aliphatic alcohol is added to the residue and the product precipitated is isolated.

5. A process as claimed in claim 5, wherein the reaction is carried out at 25 to 90° C, particularly at 50 to 70° C.

6. A process as claimed in claim 4 and 5, wherein the reaction medium is free from water.

7. A process as claimed in any of claims 4 to 6, wherein the metal cyanamide is used in excess.

8. A process as claimed in any of claims 4 to 7, wherein cyanamide is added to the reaction mixture.

9. The use of the compounds as claimed in any one of claims 1—3 for the preparation of dinitrophenylureas of the formula IV

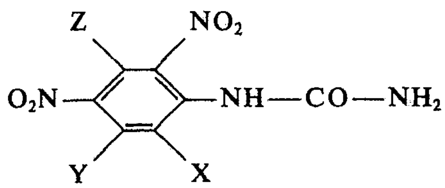

in which X, Y and Z have the meanings mentioned in claim 1, by acid hydrolysis.

10. The use as claimed in claim 9, wherein at first the corresponding cyanamide is liberated from the compounds as claimed in any one of claims 1—3.

**Revendications**

1. Composés répondant à la formule I:

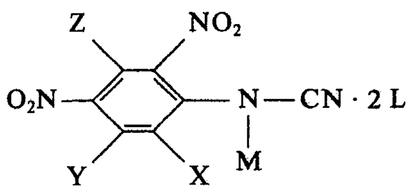

(I)

dans laquelle X représente l'hydrogène, le fluor, le chlore ou le brome, Y et Z représentent chacun l'hydrogène, un alkyle inférieur ou un alcoxy inférieur, M représente le sodium, le potassium ou un équivalent de calcium, et L représente un solvant aprotique dipolaire.

2. Composés selon la revendication 1, dans lesquels X représente l'hydrogène ou le chlore, Y et Z représentent l'hydrogène, un méthyle ou un méthoxy, M représente le sodium ou un équivalent de calcium et L représente la tétraméthylène-sulfone, la N-méthylpyrrolidone ou le diméthylsulfoxyde.

3. Composés selon la revendication 2, dans lesquels X représente le chlore, Y et Z représentent chacun l'hydrogène et M représente un équivalent de calcium.

4. Procédé de préparation des composés selon l'une quelconque des revendications 1 à 3 à partir de composés répondant à la formule II:

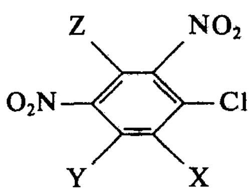

(II)

dans laquelle X, Y et Z ont les significations données à la revendication 1, et du cyanamide, dans un solvant polaire, procédé caractérisé en ce qu'on utilise le cyanamide sous la forme d'un cyanamide métallique répondant à la formule III:

$M_2N-CN$ (III)

dans laquelle M à la signification donnée à la revendication 1, et, comme solvant polaire, un solvant aprotique dipolaire, que l'on sépare dans une large mesure après la réaction, puis on ajoute au résidu un alcool aliphatique à bas poids moléculaire et on isole le produit qui a précipité.

5. Procédé selon la revendication 4, caractérisé en ce qu'on effectue la réaction à une température comprise entre 25 et 90° C, plus spécialement entre 50 et 70° C.

6. Procédé selon l'une des revendications 4 et 5, caractérisé en ce que le milieu réactionnel est anhydre.

7. Procédé selon l'une quelconque des revendications 4 à 6, caractérisé en ce que le cyanamide métallique est mis en jeu en excès.

8. Procédé selon l'une quelconque des revendications 4 à 7, caractérisé en ce qu'on ajoute du cyanamide au mélange réactionnel.

9. Application des composés selon l'une quelconque des revendications 1 à 3 à la préparation de dinitrophényl-uréesrépondant à la formule IV:

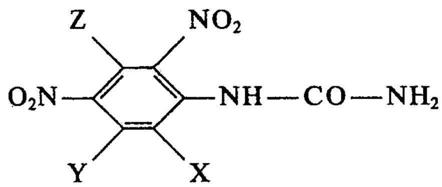

(IV)

dans laquelle X, Y et Z ont les significations données à la revendication 1, par hydrolyse acide.

10. Application selon la revendication 9, caractérisé en ce que, à partir des composés selon l'une quelconque des revendications 1 à 3, on libère d'abord le cyanamide correspondant.